# EUROPEAN PATENT APPLICATION

(11) **EP 3 190 785 A1**
(43) Date of publication of application: **12.07.2017**
(21) Application number: 15838369.5
(22) Date of filing: 02.09.2015
(51) Int. Cl.: H04N 5/243, A61B 1/04, G02B 23/24, G02B 23/26, H04N 5/225, H04N 5/374

(54) **IMAGING DEVICE AND PROCESSING DEVICE**

(30) Priority: 05.09.2014 JP 2014181305
(71) Applicant: Olympus Corporation, Hachioji-shi Tokyo 192-8507 (JP)
(72) Inventor: OGASAWARA, Kotaro, Tokyo 192-8507 (JP)
(74) Representative: Gunzelmann, Rainer
(86) International application number: PCT/JP2015/074988
(87) International publication number: WO 2016/035829

(57) **Abstract**

An endoscope system 1 includes an illumination unit 42, a light receiving unit 29a having a plurality of pixels arranged in a matrix form, a reading unit 29b for reading pixel signals from the light receiving unit 29a, for each horizontal line, an illumination controller 36 for switching an illumination state of the illumination unit 42 between a first illumination state in which illumination light is emitted, and a second illumination state in which illumination light is not emitted during the reading period, during at least part of the reading period in which the reading unit 29b reads all horizontal lines of the light receiving unit 29a, and a gain adjustment unit 32 multiplying pixel signals by different gain factors for respective horizontal lines to perform gain adjustment, the pixel signals being read during the reading period in which the illumination state of the illumination unit 42 is switched from an illumination state during a previous reading period to the other illumination state.

## Description

### Field

The present invention relates to an imaging apparatus for imaging an object to generate pixel signals, and a processing device for processing the pixel signals. Background

Endoscope systems have been used to observe the inside of a subject, in medical field. The endoscopes are commonly configured so that an elongated flexible insertion section is inserted into the subject such as a patient, illumination light supplied from a light source device is emitted from a distal end of the insertion section, reflection of the illumination light is received by an imaging unit at the distal end of the insertion section, and an in-vivo image is captured. The in-vivo image captured by the imaging unit of the endoscope appears on a display of the endoscope system, after subjected to predetermined image processing in a processing device of the endoscope system. A user, such as a physician, observes an organ of the subject based on the in-vivo image displayed on the display.

Such an endoscope system has an image sensor, and a complementary metal oxide semiconductor (CMOS) sensor may be applied as the image sensor. The CMOS sensor may generate image data by a rolling shutter method for exposure or reading of horizontal lines with a time difference.

In contrast, solid state light sources, such as an LED or a laser diode, which are used as an illumination light source, have been spread rapidly because they can adjust an electric current passing through elements to readily change the brightness, have a small size and reduced power consumption compared with a conventional lamp, and have a high response speed. However, light emitted from such a light source is known for changing in spectral characteristics depending on a current value or temperature of the light source itself. In order to avoid such a situation, control of illumination light amount (PWM control) by adjusting a light emission time of the light source is performed in addition to current control. Thus, a diaphragm mechanism or the like for adjusting an illumination light amount as in a halogen or xenon light source can be eliminated to reduce the size of a device, and an operation portion can be eliminated to reduce a failure rate.

In this endoscope system, a method for adjusting a brightness of illumination light has been proposed, in which illumination light is continuously lit when a far point is observed, and the PWM control and current control are performed during a V-blank period of a CMOS image sensor when a near point is observed (see Patent Literature 1, for example).

### Citation List

### Patent literature

Patent Literature 1: JP 5452785 B1

### Summary

### Technical problem

However, in the method described in Patent Literature 1, switching between continuous lighting and PWM control disadvantageously brings about considerable change in exposure amount for each horizontal line in the CMOS sensor, and thus, significant uneven brightness (uneven luminance) is caused, and brightness of an image displayed on the display is not smoothly changed. Furthermore, displaying a frozen still image upon switching an illumination state brings about considerable change in exposure amount for each horizontal line, and thus, horizontal uneven luminance is disadvantageously caused, and image quality cannot be maintained.

The present invention has been made in view of the foregoing, and an object of the present invention is to provide an imaging apparatus and a processing device which can maintain smooth change in brightness of a display image and image quality, even if the illumination state is changed.

### Solution to Problem

In order to solve the above described problems and achieve the object, an imaging apparatus according to the invention includes: an illumination unit configured to emit illumination light to irradiate an object; a light receiving unit having a plurality of pixels arranged on a plurality of horizontal lines, the plurality of pixels being configured to receive light from the object irradiated with the illumination light by the illumination unit, and to perform photoelectric conversion on the received light to generate pixel signals; an imaging controller configured to control the plurality of pixels of the light receiving unit to sequentially start exposure for each of the horizontal lines, and to sequentially read the pixel signals from the plurality of pixels belonging to the horizontal lines after a lapse of a predetermined exposure period from start of exposure; an illumination controller configured to control switching of an illumination state in a reading period for sequentially reading the pixel signals for each of the horizontal lines, between a first illumination state and a second illumination state different from the first illumination state; and a signal processing unit configured to perform gain adjustment by multiplying the pixel signals by different gain factors depending on the horizontal lines, the pixel signals having been read during the reading period after the illumination state of the illumination unit has been switched from the first illumination state to the second illumination state.

In the imaging apparatus according to the invention, the first illumination state is an illumination state in which the illumination light is emitted during at least part of the reading period, and the second illumination state is an illumination state in which the illumination light is not emitted during the reading period.

In the imaging apparatus according to the invention, the signal processing unit is configured to perform the gain adjustment by multiplying the pixel signals by relatively higher gain factors for later read horizontal lines, the pixel signals being read during the reading period in which the illumination state of the illumination unit is switched from the first illumination state in a previous reading period to the second illumination state.

In the imaging apparatus according to the invention, the signal processing unit is configured to perform the gain adjustment by multiplying the pixel signals by relatively lower gain factors for later read horizontal lines, the pixel signals being read during the reading period in which the illumination state of the illumination unit is switched from the second illumination state in a previous reading period to the first illumination state.

The imaging apparatus according to the invention further includes a brightness detecting unit configured to detect brightness of the object based on the read pixel signals. The illumination controller is configured to switch between the first illumination state and the second illumination state, based on the brightness of the object detected by the brightness detecting unit.

In the imaging apparatus according to the invention, the illumination controller is configured to switch between the first illumination state and the second illumination state at starting time of one frame period.

In the imaging apparatus according to the invention, the illumination controller is configured to: variably control an intensity and illumination time of the illumination light emitted from the illumination unit, during a period other than the reading period; and maintain a constant intensity of the illumination light emitted from the illumination unit, during at least part of the reading period, in the first illumination state.

In the imaging apparatus according to the invention, the illumination controller is configured to control an amount of illumination light emitted from the illumination unit such that an integrated value of the amount of light emitted from the illumination unit during one frame period immediately after switching the illumination state of the illumination unit is equal to an integrated value of the amount of light emitted from the illumination unit during next one frame period subsequent to the one frame period.

In the imaging apparatus according to the invention, the imaging apparatus is an endoscope system including: an insertion section configured to be inserted into a subject; a processing device configured to be connected to the insertion section; and a light source device configured to supply the insertion section with illumination light. The insertion section includes the light receiving unit and the imaging controller. The processing device incudes the illumination controller and the signal processing unit. The light source device includes the illumination unit.

A processing device according to the invention is a processing device for: controlling an illumination device for emitting illumination light; causing an imaging controller to sequentially start exposure for each of a plurality of horizontal lines of a light receiving unit, the light receiving unit having a plurality of pixels arranged on the plurality of horizontal lines, the plurality of pixels being configured to perform photoelectric conversion on light from an object irradiated with the illumination light; and processing pixel signals read sequentially from the plurality of pixels belonging to the horizontal lines after a lapse of a predetermined exposure period from start of exposure. The processing device includes: an illumination controller configured to control switching of an illumination state in a reading period for sequentially reading the pixel signals for each of the horizontal lines, between a first illumination state and a second illumination state different from the first illumination state; and a signal processing unit configured to perform gain adjustment by multiplying the pixel signals by different gain factors depending on the horizontal lines, the pixel signals having been read by the reading unit during the reading period after the illumination state of the illumination unit has been switched from the first illumination state to the second illumination state.

### Advantageous Effects of Invention

According to the present invention, an illumination state of an illumination unit is switched between a first illumination state and a second illumination state different from the first illumination state, pixel signals are read during a reading period after the illumination state is switched, gain adjustment is performed by multiplying the read pixel signals by different gain factors depending on horizontal lines, to eliminate uneven luminance of images. Therefore, even if the illumination state is switched, it is possible to maintain the smooth change in brightness of the display image and the image quality.

### Brief Description of Drawings

FIG. 1 is a schematic view of a configuration of an endoscope system according to an embodiment of the present invention.
FIG. 2 is a block diagram illustrating a schematic configuration of the endoscope system illustrated in FIG. 1.
FIG. 3 is a diagram illustrating emission of illumination light emitted from an illumination unit, an exposure period and a reading period of an imaging unit, and gain adjustment to pixel signals by a gain adjustment unit, in which the illumination unit, the imaging unit, and the gain adjustment unit are illustrated in FIG. 2.
FIG. 4A is a diagram illustrating an example of an image corresponding to pixel signals read by a reading unit illustrated in FIG. 2.
FIG. 4B is a diagram illustrating an example of an image corresponding to pixel signals processed in an image processing unit illustrated in FIG. 2.
FIG. 5 is a diagram illustrating an exposure period and a reading period of the imaging unit, emission of illumination light emitted from the illumination unit, and gain adjustment to pixel signals by a gain adjustment unit, in which the imaging unit, the illumination unit, and the gain adjustment unit are illustrated in FIG. 2.
FIG. 6A is a diagram illustrating an example of an image corresponding to pixel signals read by the reading unit illustrated in FIG. 2.
FIG. 6B is a diagram illustrating an example of an image corresponding to pixel signals processed in the image processing unit illustrated in FIG. 2.

### Description of Embodiments

An endoscope system will be described below as modes for carrying out the present invention (hereinafter, referred to as "embodiment (s)") . The present invention is not limited to the embodiments. The same reference signs are used to designate the same elements throughout the drawings.

### (Embodiments)

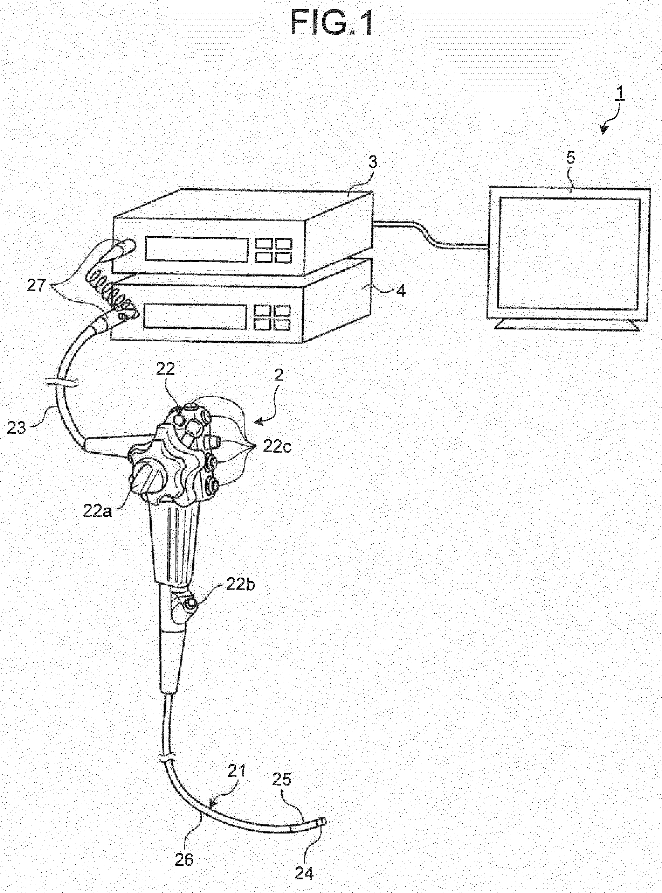

FIG. 1 is a schematic view of a configuration of an endoscope system according to an embodiment of the present invention. As illustrated in FIG. 1, an endoscope system 1 according to the embodiment includes an endoscope 2 (scope) configured to be introduced into a subject and image an inside of the subject to generate an image signal of the inside of the subject, a processing device 3 for performing predetermined image processing on the image signal captured by the endoscope 2 and controlling each unit of the endoscope system 1, a light source device 4 for generating illumination light (observation light) of the endoscope 2, and a display device 5 for displaying an image corresponding to the image signal on which the image processing has been performed by the processing device 3.

The endoscope 2 includes an insertion section 21 inserted into the subject, an operating unit 22 located on a proximal end side of the insertion section 21 and grasped by an operator, and a flexible universal cord 23 extending from the operating unit 22.

The insertion section 21 employs illumination fibers (light guide cable), an electric cable, and the like. The insertion section 21 has a distal end portion 24 having an imaging unit including a CMOS image sensor as an image sensor for imaging the inside of the subject, a bending section 25 including a plurality of bending pieces to be freely bent, and a flexible tube portion 26 provided on a proximal end side of the bending section 25 and having flexibility. The distal end portion 24 is provided with an illumination unit for illuminating the inside of the subject through an illumination lens, an imaging unit for imaging the inside of the subject, an opening (not illustrated) communicating with a treatment tool channel, and an air/water feeding nozzle (not illustrated).

The operating unit 22 has a bending knob 22a for bending the bending section 25 vertically and horizontally, a treatment tool insertion section 22b through which a treatment tool such as biopsy forceps or a laser scalpel is configured to be inserted into a body cavity of the subject, and a plurality of switch portions 22c for operating peripheral devices such as the processing device 3, the light source device 4, an air feeding device, a water feeding device, and a gas feeding device. The treatment tool inserted through the treatment tool insertion section 22b is exposed from an opening at a distal end of the insertion section 21 through the treatment tool channel provided therein.

The universal cord 23 employs illumination fibers, an electric cable, and the like. The universal cord 23 has a connector 27 bifurcated at a proximal end to be removably mounted to the processing device 3 and the light source device 4. The universal cord 23 transmits the image signal captured by the imaging unit provided at the distal end portion 24, to the processing device 3 through the connector 27. The universal cord 23 transmits illumination light emitted from the light source device 4, to the distal end portion 24 through the connector 27, the operating unit 22, and the flexible tube portion 26.

The processing device 3 performs predetermined image processing on the imaging signal of the inside of the subject, which has been obtained by the imaging unit located at the distal end portion 24 of the endoscope 2, and input through the universal cord 23. The processing device 3 controls each unit of the endoscope system 1 based on various instruction signals transmitted from the switch portions 22c in the operating unit 22 of the endoscope 2, through the universal cord 23.

The light source device 4 employs a light source, a condenser lens, or the like for emitting white light. The light source device 4 supplies the endoscope 2 connected through the connector 27 and the illumination fibers of the universal cord 23 with white light from a white light source to illuminate the subject as an object.

The display device 5 employs a liquid crystal or organic electro luminescence (EL) display or the like. The display device 5 displays, through an image cable, various information including an image corresponding to a display image signal subjected to predetermined image processing by the processing device 3. Thus, the operator can operate the endoscope 2, while viewing an image (in-vivo image) displayed on the display device 5, and a desired position in the subject can be observed and characteristics thereof can be determined.

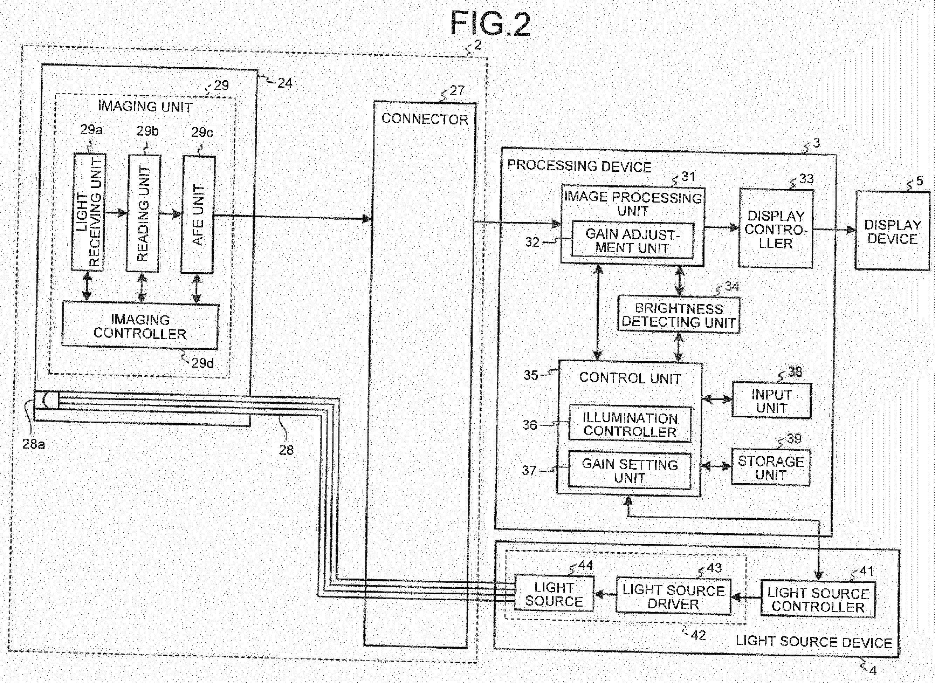

A configuration of the endoscope system 1 illustrated in FIG. 1 will be described next. FIG. 2 is a block diagram illustrating a schematic configuration of the endoscope system 1 illustrated in FIG. 1.

The endoscope 2 has an imaging unit 29 at the distal end portion 24. The imaging unit 29 includes a light receiving unit 29a for generating pixel signals representing the inside of the subject, from an optical image focused on a light receiving surface, a reading unit 29b, an analog front end unit (hereinafter referred to as "AFE unit") 29c, and an imaging controller 29d. Note that an optical system (not illustrated), such as an objective lens, is disposed on a light receiving surface side of the light receiving unit 29a.

In the light receiving unit 29a, a plurality of pixels is arranged on the light receiving surface. Each of the pixels receives light from the object illuminated by the light source device 4, photoelectrically converts the received light, and generates a pixel signal. In the light receiving unit 29a, the plurality of pixels is arranged in a matrix form. In the light receiving unit 29a, a plurality of pixel rows (horizontal lines) each having two or more pixels arranged along a horizontal direction is arranged in a vertical direction. The light receiving unit 29a generates the pixel signals representing the inside of the subject from the optical image focused on the light receiving surface.

The reading unit 29b performs exposure of the plurality of pixels in the light receiving unit 29a, and reading of the pixel signals from the plurality of pixels. The light receiving unit 29a and the reading unit 29b include for example the CMOS image sensor, and can perform exposure and reading for each horizontal line. The reading unit 29b can also generate the pixel signals by a rolling shutter method. The rolling shutter method performs imaging operation of exposure and reading, from a top horizontal line, and performs resetting of electrical charge, exposure, and reading for each horizontal line with a time difference. Thus, when the imaging unit 29 employs the rolling shutter method, exposure timing and read timing for each horizontal line are different even in one imaging time (frame).

The AFE unit 29c performs noise removal, A/D conversion, or the like on an electrical signal of a pixel signal read by the reading unit 29b. The AFE unit 29c performs reduction of a noise component included in the electrical signal (analog), adjustment of an amplification rate (gain) of the electrical signal to maintain an output level, and A/D conversion of the analog electrical signal.

The imaging controller 29d controls various operations of the light receiving unit 29a, the reading unit 29b, and the AFE unit 29c of the imaging unit 29, according to a control signal received from the processing device 3. An image signal (digital) generated by the imaging unit 29 is output to the processing device 3 through a signal cable not illustrated or the connector 27.

Next, the processing device 3 will be described. The processing device 3 includes an image processing unit 31, a display controller 33, a brightness detecting unit 34, a control unit 35, an input unit 38, and a storage unit 39.

The image processing unit 31 performs predetermined signal processing on the pixel signals (image signal) of the plurality of pixels read by the reading unit 29b of the imaging unit 29. The image processing unit 31 performs, on the pixel signals, image processing such as optical black subtraction, gain adjustment, or white balance (WB) adjustment. When the image sensor has a Bayer array, the image processing unit 31 performs, on the image signal, image processing such as synchronization, color matrix calculation, gamma correction, color reproduction, or edge enhancement. The image processing unit 31 includes a gain adjustment unit 32 for performing gain adjustment.

The display controller 33 generates the display image signal to be displayed on the display device 5, from the image signal processed by the image processing unit 31, and outputs the display image signal to the display device 5. The display image signal output to the display device 5 is for example a digital signal having an SDI, DVI, or HDMI (registered trade mark) format. Alternatively, the display controller 33 may convert the display image signal from the digital signal to the analog signal, then change image data of the converted analog signal to have a format of a high vision system or the like, and output the image data to the display device 5.

The brightness detecting unit 34 detects a brightness of the object, based on the pixel signals read by the reading unit 29b. The brightness detecting unit 34 obtains for example sample image data from the image processing unit 31, detects a brightness level corresponding to each pixel, and outputs the detected brightness level to the control unit 35.

The control unit 35 employs a CPU or the like. The control unit 35 controls processing operation of each unit of the processing device 3. The control unit 35 transfers instruction information, data, or the like to each component of the processing device 3 to control the operation of the processing device 3. The control unit 35 is connected to the imaging unit 29 and the light source device 4 through cables. The control unit 35 has an illumination controller 36 and a gain setting unit 37.

The illumination controller 36 controls switching of an illumination state of an illumination unit 42 described later, between a first illumination state and a second illumination state. In the first illumination state, illumination light is emitted during at least part of a reading period of one frame period in which the reading unit 29b reads all of the horizontal lines of the light receiving unit 29a. In the second illumination state, illumination light is not emitted during the reading period. The illumination controller 36 performs switching between the first illumination state and the second illumination state, based on the brightness of the object detected by the brightness detecting unit 34. The illumination controller 36 performs switching between the first illumination state and the second illumination state at starting time of one frame period.

In the first illumination state, the illumination controller 36 maintains a constant intensity of illumination light emitted from the illumination unit 42, during at least part of the reading period. That is, the illumination controller 36 controls the illumination state of the illumination unit 42 not to cause temporal change in amount of light, during at least part of a single reading period. The illumination controller 36 variably controls an intensity and illumination time of the illumination light emitted from the illumination unit 42, during a period other than the reading period. In other words, the illumination controller 36 performs PWM control of the intensity and illumination time of the illumination light emitted from the illumination unit 42 of the light source device 4, during a period other than the reading period. Thus, the illumination controller 36 controls an amount of illumination light emitted from the illumination unit 42 such that an integrated value of the amount of light emitted from the illumination unit 42 during one frame period immediately after switching the illumination state of the illumination unit 42 is equal to an integrated value of the amount of light emitted from the illumination unit 42 during next one frame period subsequent to the one frame period. Thus, a curve indicating an amount of illumination light emitted from the illumination unit 42 can be continuously changed. The illumination controller 36 sets the amount, emission timing, or the like of illumination light emitted from the illumination unit 42, based on the brightness of the object included in the image signal, which is detected by the brightness detecting unit 34, and outputs a light-controlled signal including the set conditions to the light source device 4.

The gain setting unit 37 calculates a gain factor, based on the brightness level detected by the brightness detecting unit 34, and outputs the calculated gain factor to the gain adjustment unit 32. In the gain setting unit 37, different gain factors for respective horizontal lines are set to the pixel signals read by the reading unit 29b during a reading period in which the illumination state of the illumination unit 42 is switched from an illumination state during a previous reading period to the other illumination state. Thus, in the gain adjustment unit 32, the pixel signals read by the reading unit 29b during a reading period in which the illumination state of the illumination unit 42 is switched from an illumination state during a previous reading period to the other illumination state, are multiplied by the different gain factors for respective horizontal lines, and thus gain adjustment is performed.

The input unit 38 employs an operation device such as a mouse, a keyboard, or a touch panel, and receives input of various instruction information for the endoscope system 1. Specifically, the input unit 38 receives input of subject information (e.g., ID, date of birth, name, or the like), identification information of the endoscope 2 (e.g., ID or examination item), and various instruction information such as examination contents.

The storage unit 39 employs a volatile memory or a non-volatile memory, and stores various programs for operating the processing device 3 and the light source device 4. The storage unit 39 temporarily stores information being processed by the processing device 3. The storage unit 39 stores the pixel signals read by the reading unit 29b in frames. The storage unit 39 may use a memory card or the like mounted from outside the processing device 3.

Next, the light source device 4 will be described. The light source device 4 has a light source controller 41, and the illumination unit 42 provided with a light source driver 43 and a light source 44.

The light source controller 41 controls emission of illumination light from the illumination unit 42, under the control of the illumination controller 36. The light source driver 43 supplies the light source 44 with predetermined power, under the control of the light source controller 41. The light source 44 employs a light source such as a white LED for emitting white light, and an optical system such as a condenser lens. The light source 44 generates illumination light for supplying the endoscope 2. The light emitted from the light source 44 is transmitted through a light guide cable 28 to an illumination window 28a at the distal end portion 24 of the insertion section 21, through the connector 27 and the universal cord 23, and the light illuminates the object. Note that, the imaging unit 29 is disposed in the vicinity of the illumination window 28a.

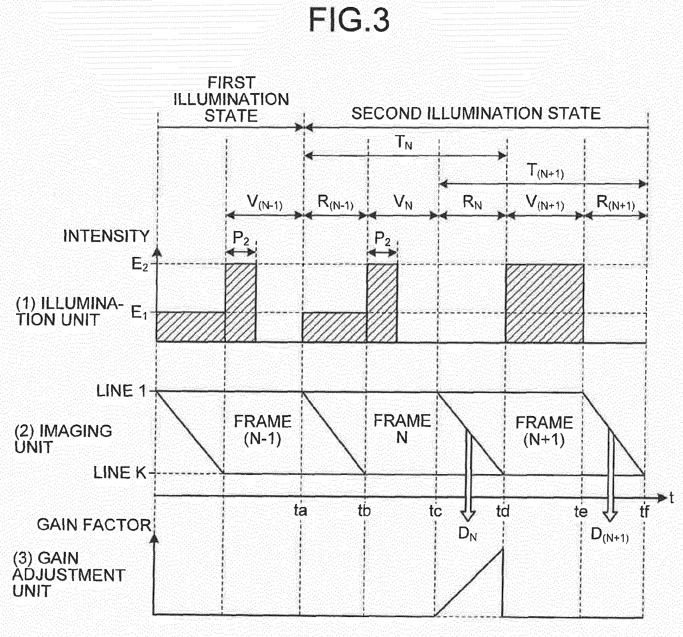

First, the illumination state of the illumination unit 42 will be described. The illumination state of the illumination unit 42 is switched from the first illumination state to the second illumination state to gradually reduce the amount of illumination light. FIG. 3 is a diagram illustrating illumination processing of illumination light emitted from the illumination unit 42, an exposure period and a reading period of the imaging unit 29, and the gain adjustment by the gain adjustment unit 32 for the pixel signals read by the reading unit 29b, in the endoscope system 1. A timing chart of illumination timing and the intensity of illumination light emitted from the illumination unit 42 is illustrated in (1) of FIG. 3. A timing chart of the exposure period and the reading period in the imaging unit 29 is illustrated in (2) of FIG. 3. A schematic diagram of the gain factors actually used for gain adjustment by the gain adjustment unit 32 is chronologically illustrated in (3) of FIG. 3. In (3) of FIG. 3, a time axis corresponding to the gain adjustment by the gain adjustment unit 32 is shifted so that the gain factors actually used for the gain adjustment by the gain adjustment unit 32, and read timing for reading the horizontal lines subjected to the gain adjustment are positioned on the same line.

When the imaging unit 29 employs the rolling shutter method in which exposure timing and read timing are changed for each horizontal line, even if the pixel signals are in the same frame, the exposure timing and the read timing are different in respective horizontal lines, as illustrated in (2) of FIG. 3. In a frame N, a frame period T_{N} from time ta to time td includes a period from time tc to time td defined as a reading period R_{N} in which the reading unit 29b reads pixel signals D_{N} of all horizontal lines of the light receiving unit 29a. A period from time ta to time td other than the reading period R_{N}, includes a period from time ta to time tb overlapping with a reading period R_{(N-1)} for a previous frame (N-1), in which exposure is started sequentially from the top horizontal line in the frame (N-1) where reading is finished, and a period from time tb to time tc defined as an entire simultaneous exposure period (V-blank period) V_{N} for all lines in which all horizontal lines of the light receiving unit 29a are simultaneously exposed. In a frame (N+1), a frame period T_{(N+1)} from time tc to time tf includes a period from time te to time tf defined as a reading period R_{(N+1)}, a period from time tc to time te other than the reading period R_{(N+1)} includes a period from time tc to time td which overlapping with the reading period R_{N} for the frame N, in which exposure is started sequentially from the top horizontal line in the frame N where reading is finished, and a period from time td to time te defined as an entire simultaneous exposure period V_{(N+1)} for all lines.

In the reading period, the illumination state of the illumination unit 42 is controlled by the illumination controller 36 so that the illumination unit 42 has the first illumination state in which illumination light having a certain intensity is emitted, or the second illumination state in which illumination light is not emitted. In an example of FIG. 3, the first illumination state is switched to the second illumination state at starting time (time ta) of the frame N. In the reading period R_{(N-1)} for the frame (N-1), the illumination unit 42 emits for example illumination light having an intensity E₁ (first illumination state), and in the reading period R_{N} for the frame N and the reading period R_{(N+1)} for the frame (N+1), illumination light is not emitted (second illumination state).

An entire simultaneous exposure period V_{N} for all lines in the frame period T_{N}, the illumination unit 42 emits illumination light having an intensity E₂ only during a period P₂ in the entire simultaneous exposure period V_{N} for all lines, similarly to an entire simultaneous exposure period V_{(N-1)} for all lines in the frame (N-1). In this configuration, in order to achieve continuous control of light amount between the frame N having the switched illumination state and the frame (N+1) subsequent to the frame N, the illumination controller 36 controls the amount of illumination light emitted from the illumination unit 42 such that an integrated value of the amount of light emitted from the illumination unit 42 during the frame period T_{N} is equal to an integrated value of the amount of light emitted from the illumination unit 42 during the frame period T_{(N+1)}. Specifically, the illumination controller 36 causes the illumination unit 42 to emit illumination light having an intensity E₂ over the entire simultaneous exposure period V_{(N+1)} for all lines in the frame (N+1) so that an integrated value L_{(N+1)} (= V_{(N+1)} × E₂) of the amount of light emitted from the illumination unit 42 during the frame period T_{(N+1)}, and an integrated value L_{N} (= R_{(N-1)} × E₁ + P₂ × E₂) of the amount of light emitted from the illumination unit 42 during the frame period T_{N} are equal to each other. In frames subsequent to the frame (N+1), the illumination controller 36 reduces for example the illumination time during each entire exposure period for all lines, or the intensity of illumination light to gradually reduce the amount of illumination light emitted to the object.

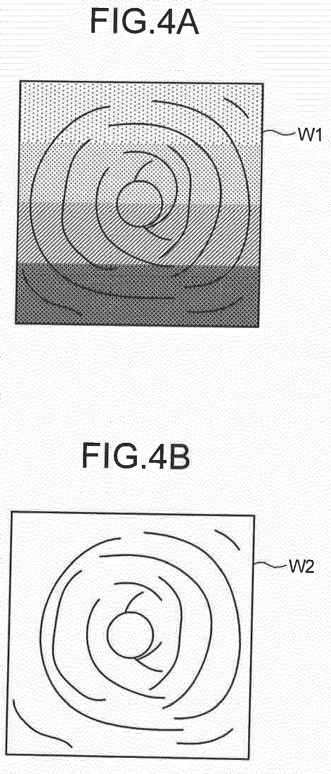

FIG. 4A is a diagram illustrating an example of an image corresponding to pixel signals read by the reading unit 29b. FIG. 4B is a diagram illustrating an example of an image corresponding to pixel signals processed in a signal processing unit 31. As illustrated in (2) of FIG. 3, an image in the frame N is exposed to light having the same amount in any horizontal line, in the entire simultaneous exposure period V_{N} for all lines, but in the reading period R_{(N-1)}, exposure amount differs in respective horizontal lines, and the exposure amount is reduced from the top horizontal line to the bottom horizontal line. Thus, as illustrated in FIG. 4A, when the reading unit 29b reads the pixel signals from each horizontal line of the light receiving unit 29a, thus obtained image W1 is gradually darkened from an upper part to a lower part, and uneven luminance is generated.

Consequently, the gain setting unit 37 obtains an exposure time in each horizontal line of the frame N, from the illumination controller 36, and sets the different gain factors for respective horizontal lines according to the obtained exposure time in each horizontal line. In an example of FIG. 3, the gain setting unit 37 sets a relatively higher gain factor to a horizontal line read later, for the pixel signals D_{N} of the frame N read by the reading unit 29b during the reading period R_{N}. That is, the gain setting unit 37 sets the gain factor to be relatively gradually increased as line number increases from the top horizontal line 1 toward the bottom horizontal line K. Consequently, as illustrated in (3) of FIG. 3, in the gain adjustment unit 32, the pixel signals of the frame N are multiplied by a relatively higher gain factor as a horizontal line is read later, and thus the gain adjustment is performed. This gain adjustment performed by the gain adjustment unit 32 generates a corrected image W2 (see FIG. 4B) in which uneven luminance is corrected. Note that all horizontal lines are exposed to illumination light having a certain intensity E₂ during the entire simultaneous exposure period V_{(N+1)} for all lines, no variation is caused in exposure amount between the horizontal lines, and thus, the gain factor used in the gain adjustment unit 32 is the same in any horizontal line, for the pixel signals D_{(N+1)} of the frame (N+1) read during the reading period R_{(N+1)}.

As descried above, in the gain adjustment unit 32, the pixel signals are multiplied by a relatively higher gain factor as a horizontal line is read later, and thus, the gain adjustment is performed to correct uneven luminance. Here, the pixel signals are read by the reading unit 29b during the reading period in which the illumination state of the illumination unit 42 is switched from the first illumination state being an illumination state in the previous reading period to the second illumination state.

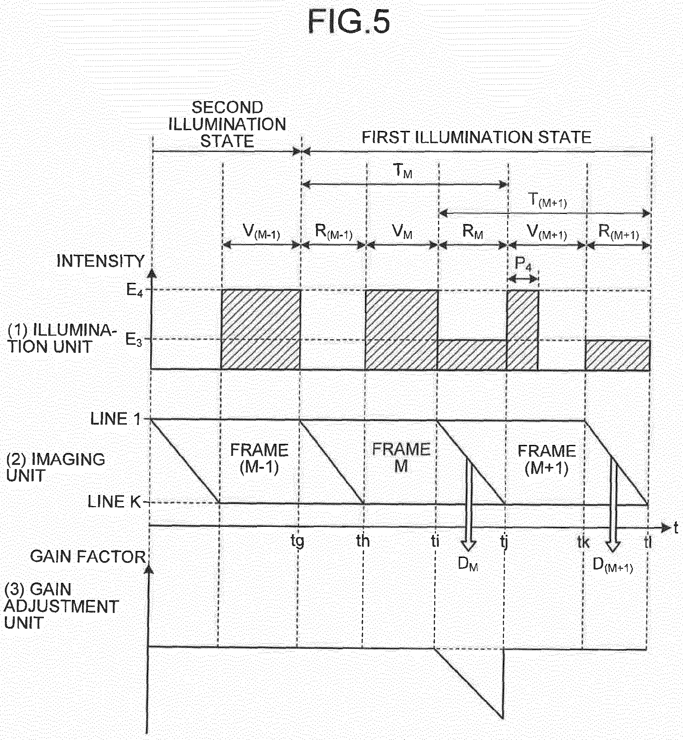

Next, the illumination state of the illumination unit 42 will be described, in which the illumination state of the illumination unit 42 is switched from the second illumination state to the first illumination state to gradually increase the amount of illumination light. FIG. 5 is a diagram illustrating illumination processing of illumination light emitted from the illumination unit 42, an exposure period and a reading period of the imaging unit 29, and the gain adjustment by the gain adjustment unit 32 to the pixel signals read by the reading unit 29b, in this configuration. A timing chart of illumination timing and the intensity of illumination light emitted from the illumination unit 42 is illustrated in (1) of FIG. 5. A timing chart of the exposure period and the reading period in the imaging unit 29 is illustrated in (2) of FIG. 5. A schematic diagram of the gain factors actually used for the gain adjustment by the gain adjustment unit 32 is chronologically illustrated in (3) of FIG. 5. Similarly to (3) of FIG. 3, also in (3) of FIG. 5, a time axis corresponding to the gain adjustment by the gain adjustment unit 32 is shifted so that the gain factors actually used for the gain adjustment by the gain adjustment unit 32, and the read timing for reading the horizontal lines subjected to the gain adjustment are positioned on the same line.

In a frame M, a frame period T_{M} from time tg to time tj includes a period from time ti to time tj defined as a reading period R_{M}, a period from time tg to time th overlapping with a reading period R_{(M-1)} for a previous frame (M-1) in which exposure is started sequentially from the top horizontal line in the frame (M-1) where reading is finished, and a period from time th to time ti defined as an entire simultaneous exposure period V_{M} for all lines in which all horizontal lines of the light receiving unit 29a are simultaneously exposed. In a frame (M+1), a frame period T_{(M+1)} from time ti to time tl includes a period from time tk to time tl defined as a reading period R_{(M+1)}, a period from time ti to time tj overlapping with the reading period R_{M} in which exposure is started sequentially from the top horizontal line in the frame M where reading is finished, and a period from time tj to time tk defined as an entire simultaneous exposure period V_{(M+1)} for all lines.

In an example of FIG. 5, the illumination unit 42 is switched from the second illumination state to the first illumination state, at starting time (time tg) of the frame M. That is, in the reading period R_{(M-1)} for the frame (M-1), the illumination unit 42 is switched from the second illumination state in which illumination light is not emitted, to the first illumination state in which illumination light having an intensity E₃ is emitted in the reading period R_{M} for the frame M and the reading period R_{(M+1)} for the frame (M+1). The entire simultaneous exposure period V_{M} for all lines in the frame period T_{M}, the illumination unit 42 emits illumination light having an intensity E₄, similarly to an entire simultaneous exposure period V_{(M-1)} for all lines in the frame (M-1). In this configuration, in order to achieve continuous control of light amount between the frame M having the switched illumination state and the frame (M+1) subsequent to the frame M, the illumination controller 36 causes the illumination unit 42 to emit illumination light having an intensity E₄ in a partial period P₄ of the entire simultaneous exposure period V_{(M+1)} for all lines in the frame (M+1) so that an integrated value L_{(M+1)} (= R_{M} × E₃ + P₄ × E₄ + R_{(M+1)} × E₃) of the amount of light emitted from the illumination unit 42 during the frame period T_{(M+1)}, and an integrated value L_{M} (= V_{M} × E₄ + R_{M} × E₃) of the amount of light emitted from the illumination unit 42 during the frame period T_{M} are equal to each other. In frames subsequent to the frame (M+1), for example, the illumination controller 36 extends the illumination time during each entire exposure period for all lines, enhances the intensity of the illumination light, or enhances the intensity of the illumination light during the reading period to gradually increase the amount of illumination light emitted to the object.

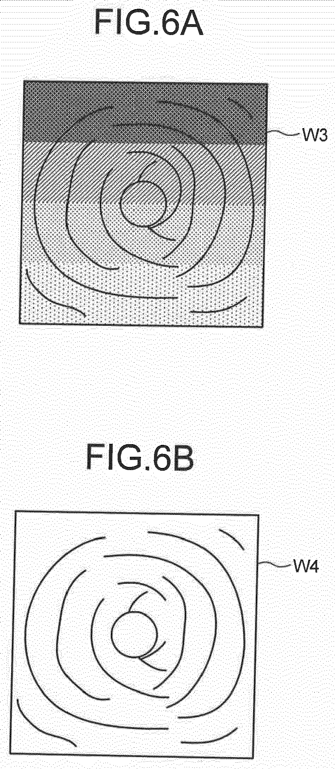

FIG. 6A is a diagram illustrating an example of an image corresponding to pixel signals read by the reading unit 29b. FIG. 6B is a diagram illustrating an example of an image corresponding to pixel signals processed in the signal processing unit 31. As illustrated in (2) of FIG. 5, an image in the frame M has an exposure amount increasing from the top horizontal line to the bottom horizontal line in the reading period R_{M}, and thus when the reading unit 29b reads the pixel signals from each horizontal line of the light receiving unit 29a, thus obtained image W3 (see FIG. 6A) is gradually brightened from an upper part to a lower part, and uneven luminance is generated.

Thus, the gain setting unit 37 sets the gain factor to be relatively gradually reduced relative to line number increasing from the top horizontal line 1 to the bottom horizontal line K, for pixel signals D_{M} of the frame M read by the reading unit 29b during the reading period R_{M}. Consequently, as illustrated in (3) of FIG. 5, in the gain adjustment unit 32, the pixel signals of the frame M are multiplied by a relatively lower gain factor as a horizontal line read later, and thus the gain adjustment is performed to generate a corrected image W4 (see FIG. 6B) in which uneven luminance is corrected. Note that illumination processing is controlled so that the integrated value of the amount of light emitted during the frame period T_{(M+1)} has the same value in any horizontal line, and thus, the gain factor used in the gain adjustment unit 32 is the same in any horizontal line, for the pixel signals D_{(M+1)} of the frame (M+1) read during the reading period R_{(M+1)}.

As descried above, in the gain adjustment unit 32, the pixel signals are multiplied by a relatively lower gain factor as a horizontal line is read later, and thus, the gain adjustment is performed to correct uneven luminance. Here, the pixel signals are read by the reading unit 29b during a reading period in which the illumination state of the illumination unit 42 is switched from the first illumination state being an illumination state during a previous reading period to the second illumination state.

As described above, according to an embodiment, the pixel signals read by the reading unit 29b during a reading period in which the illumination state of the illumination unit 42 is switched from an illumination state during a previous reading period to the other illumination state, are multiplied by the different gain factors for respective horizontal lines, and uneven luminance of the image is eliminated. Therefore, even if the illumination state is switched, the smooth change in brightness of the display image and the image quality can be maintained.

Note that in the embodiment, the first illumination state being a state in which illumination light is emitted over the reading period has been described, but, as a matter of course, a state in which illumination light is emitted only during at least a partial period of the reading period may be employed. Furthermore, the illumination controller 36 may perform switching between the first illumination state and the second illumination state not only at the starting time of one frame period, but at a desired time. A frame to be subjected to the gain adjustment or horizontal lines to which different gain factors are to be set is different depending on time at which the illumination controller 36 performs switching between the first illumination state and the second illumination state. Thus the gain setting unit 37 preferably determines the frame to be subjected to the gain adjustment or the horizontal lines to which different gain factors are to be set, according to time at which the illumination controller 36 performs switching between the first illumination state and the second illumination state, and the read timing at which the reading unit 29b reads the horizontal lines. Furthermore, in the embodiment, illumination control has been exemplified which equalizes the integrated values of the amount of illumination light during frame periods, between a frame having a switched illumination state and a subsequent frame, but, as a matter of course, the illumination control is not particularly limited to the above, and the integrated values of the amount of illumination light during frame periods may be different between the frame having a switched illumination state and the subsequent frame.

In addition, programs to be executed for various processing executed in the processing device 3 according to the embodiment and the other components may be provided to be recorded in a computer-readable recording medium such as a CD-ROM, flexible disk, CD-R, digital versatile disk (DVD), in an installable format file or in an executable format file, or the programs may be stored on a computer connected to a network such as the Internet, and provided to be downloaded through the network. Furthermore, the programs may be provided or distributed through the network such as the Internet.

### Reference Signs List

- 1: ENDOSCOPE SYSTEM
- 2: ENDOSCOPE
- 3: PROCESSING DEVICE
- 4: LIGHT SOURCE DEVICE
- 5: DISPLAY DEVICE
- 21: INSERTION SECTION
- 22: OPERATING UNIT
- 23: UNIVERSAL CORD
- 24: DISTAL END PORTION
- 25: BENDING SECTION
- 26: FLEXIBLE TUBE PORTION
- 27: CONNECTOR
- 28: LIGHT GUIDE CABLE
- 29: IMAGING UNIT
- 29a: LIGHT RECEIVING UNIT
- 29b: READING UNIT
- 29c: AFE UNIT
- 29d: IMAGING CONTROLLER
- 31: IMAGE PROCESSING UNIT
- 32: GAIN ADJUSTMENT UNIT
- 33: DISPLAY CONTROLLER
- 34: BRIGHTNESS DETECTING UNIT
- 35: CONTROL UNIT
- 36: ILLUMINATION CONTROLLER
- 37: GAIN SETTING UNIT
- 38: INPUT UNIT
- 39: STORAGE UNIT
- 41: LIGHT SOURCE CONTROLLER
- 42: ILLUMINATION UNIT
- 43: LIGHT SOURCE DRIVER
- 44: LIGHT SOURCE

## Claims

1. An imaging apparatus comprising:
an illumination unit configured to emit illumination light to irradiate an object;
a light receiving unit having a plurality of pixels arranged on a plurality of horizontal lines, the plurality of pixels being configured to receive light from the object irradiated with the illumination light by the illumination unit, and to perform photoelectric conversion on the received light to generate pixel signals;
an imaging controller configured to control the plurality of pixels of the light receiving unit to sequentially start exposure for each of the horizontal lines, and to sequentially read the pixel signals from the plurality of pixels belonging to the horizontal lines after a lapse of a predetermined exposure period from start of exposure;
an illumination controller configured to control switching of an illumination state in a reading period for sequentially reading the pixel signals for each of the horizontal lines, between a first illumination state and a second illumination state different from the first illumination state; and
a signal processing unit configured to perform gain adjustment by multiplying the pixel signals by different gain factors depending on the horizontal lines, the pixel signals having been read during the reading period after the illumination state of the illumination unit has been switched from the first illumination state to the second illumination state.

2. The imaging apparatus according to claim 1, wherein
the first illumination state is an illumination state in which the illumination light is emitted during at least part of the reading period, and
the second illumination state is an illumination state in which the illumination light is not emitted during the reading period.

3. The imaging apparatus according to claim 2, wherein
the signal processing unit is configured to perform the gain adjustment by multiplying the pixel signals by relatively higher gain factors for later read horizontal lines, the pixel signals being read during the reading period in which the illumination state of the illumination unit is switched from the first illumination state in a previous reading period to the second illumination state.

4. The imaging apparatus according to claim 2, wherein
the signal processing unit is configured to perform the gain adjustment by multiplying the pixel signals by relatively lower gain factors for later read horizontal lines, the pixel signals being read during the reading period in which the illumination state of the illumination unit is switched from the second illumination state in a previous reading period to the first illumination state.

5. The imaging apparatus according to claim 1, further comprising a brightness detecting unit configured to detect brightness of the object based on the read pixel signals, wherein
the illumination controller is configured to switch between the first illumination state and the second illumination state, based on the brightness of the object detected by the brightness detecting unit.

6. The imaging apparatus according to claim 1, wherein
the illumination controller is configured to switch between the first illumination state and the second illumination state at starting time of one frame period.

7. The imaging apparatus according to claim 6, wherein the illumination controller is configured to:
variably control an intensity and illumination time of the illumination light emitted from the illumination unit, during a period other than the reading period; and
maintain a constant intensity of the illumination light emitted from the illumination unit, during at least part of the reading period, in the first illumination state.

8. The imaging apparatus according to claim 6, wherein
the illumination controller is configured to control an amount of illumination light emitted from the illumination unit such that an integrated value of the amount of light emitted from the illumination unit during one frame period immediately after switching the illumination state of the illumination unit is equal to an integrated value of the amount of light emitted from the illumination unit during next one frame period subsequent to the one frame period.

9. The imaging apparatus according to claim 1, wherein
the imaging apparatus is an endoscope system including: an insertion section configured to be inserted into a subject; a processing device configured to be connected to the insertion section; and a light source device configured to supply the insertion section with illumination light,
the insertion section includes the light receiving unit and the imaging controller,
the processing device incudes the illumination controller and the signal processing unit, and
the light source device includes the illumination unit.

10. A processing device for: controlling an illumination device for emitting illumination light; causing an imaging controller to sequentially start exposure for each of a plurality of horizontal lines of a light receiving unit, the light receiving unit having a plurality of pixels arranged on the plurality of horizontal lines, the plurality of pixels being configured to perform photoelectric conversion on light from an object irradiated with the illumination light; and processing pixel signals read sequentially from the plurality of pixels belonging to the horizontal lines after a lapse of a predetermined exposure period from start of exposure, the processing device comprising:
an illumination controller configured to control switching of an illumination state in a reading period for sequentially reading the pixel signals for each of the horizontal lines, between a first illumination state and a second illumination state different from the first illumination state; and
a signal processing unit configured to perform gain adjustment by multiplying the pixel signals by different gain factors depending on the horizontal lines, the pixel signals having been read by the reading unit during the reading period after the illumination state of the illumination unit has been switched from the first illumination state to the second illumination state.
